# DEMANDE DE BREVET EUROPEEN

(11) **EP 2 329 766 A1**
(43) Date de publication de la demande: **08.06.2011**
(21) Numéro de dépôt: 10193443.8
(22) Date de dépôt: 02.12.2010
(51) Int. Cl.: A61B 5/0402, G01B 3/16, G01B 3/20

(54) **Instrument pour la lecture d'un tracé ECG**

(30) Priorité: 03.12.2009 CH 18612009
(71) Demandeur: Stanga, Carlo, 6533 Lumino (CH)
(72) Inventeur: Stanga, Carlo, 6533 Lumino (CH)
(74) Mandataire: Zardi, Marco

(57) **Abrégé**

Instrument de support pour la lecture d'un tracé ECG imprimé sur papier, réalisé de préférence comme un pied à coulisse ou un compas, qui contient un premier élément de référence (13, 34) et un deuxième élément de référence (14, 35) associés respectivement à un premier composant (11, 31) et à un deuxième composant (12, 32) de l'instrument déplaçables entre eux de façon que la distance linéaire entre le premier élément de référence et le deuxième élément de référence est réglable, et un système de mesure intégré de type numérique ou analogique pour permettre la lecture d'au moins une grandeur caractéristique d'un tracé ECG, par exemple la fréquence cardiaque ou l'intervalle de temps en ms.

## Description

### Domaine de l'invention

L'invention concerne un dispositif technique pour aider à la lecture d'un électrocardiogramme (ECG). En particulier l'invention concerne un instrument pour le calcul de grandeurs caractéristiques à partir d'un tracé ECG imprimé sur papier.

### État de la technique

L'électrocardiogramme (ECG), il est bien connu, produit un graphique su papier qui montre l'activité du coeur, relevée via des électrodes appliquées sur différentes parties du corps. Le graphique électrocardiographe normal est composé de trois ondes positives et deux négatives qui correspondent aux différentes activités de systole et diastole des oreillettes et des ventricules du myocarde. La vitesse d'impression (alimentation du papier) couramment utilisé est 25 mm/s ou 50 mm/s. Le papier est généralement millimétré.

Le tracé périodique d'un ECG peut être divisé en cinq parties. L'onde montre des pics que, dans la littérature du secteur, sont appelé avec les lettres P, Q, R, S et T. La période commence avec une onde de dimension réduite, l'onde P, qui est le résultat de la contraction des oreillettes, qui nous donne l'indication du temps de propagation de l'impulse dans les deux oreillettes (systole auriculaire). Suit un trait plat sans activité, le segment PQ, qui indique le passage du stimulus depuis les oreillettes aux ventricules. Ensuite on trouve le complexe QRS, composé par l'onde Q, courte et vers le bas, l'onde R typiquement grande et étroite, et une petite onde S, elle aussi vers le bas; le complexe est le représentation de la systole ventriculaire avec l'arrivée du stimulus aux ventricules (onde Q) et l'extension au reste du tissu (onde R et S). Le complexe QRS donne des indications pour des éventuelles arythmies, fibrillations et peut être utile en cas d'infarctus. L'intervalle ST qui suit l'onde S et comprend l'onde T peut discerner des problèmes ischémiques, en étant le temps avec lequel les ventricules se contractent et ensuite (avec onde T) se relaxent. L'onde T permet d'avoir des indications sur l'hypertrophie cardiaque, l'infarctus e l'ischémie.

Dans la pratique quotidienne du cardiologue ou médecin en généraliste, il est nécessaire de déterminer un ensemble de variables caractéristiques à partir du tracé lui-même. Par exemple, commune est la nécessité de mesurer la fréquence cardiaque, la régularité du rythme et la longueur du temps (en millisecondes, ms) des intervalles spécifiques (ST, QRS ...).

La lecture du graphique ECG et la détermination de ces grandeurs, dans l'art connue, impliquent toujours une procédure essentiellement manuelle.

Une première approche est le calcul manuel. En comptant les carrés de papier millimétré entre deux périodes consécutives du rythme et en faisant une division, il est possible de déterminer la fréquence d'un intervalle (fréquence instantanée). Par exemple: avec une vitesse d'impression de 25 mm / s, un millimètre sur le graphique correspond à 40 ms, si l'intervalle entre deux mesures consécutives est de 20 mm sur le papier, il correspond à 400 ms, la fréquence de battements par minute (bpm) peut alors être calculée comme 60.000/400 = 150 bpm. Cette méthode, il est facile à comprendre, est plutôt lourde et peu pratique.

Pour éviter le calcul manuel, on connait l'utilisation d'un instrument composé d'une réglette transparente, avec une échelle graduée et normalisée en fonction de la vitesse d'impression (par exemple 25 mm/s). La réglette est superposée sur le graphique, en alignant le début de l'échelle (la ligne zéro) avec un point précis du tracé, par exemple une onde R. La lecture de l'échelle graduée, correspondant à l'onde R suivante, fournit la valeur de la fréquence. Cette approche est évidemment plus pratique et plus rapide que le calcul manuel, cependant il reste une marge d'erreur tant dans le positionnement de la réglette que dans la lecture. En outre, la lecture est plus difficile, et avec la plus grande marge d'erreur pour les fréquences les plus élevées, par exemple supérieures à 150 bpm, où l'échelle est très dense.

En outre la réglette ne permet pas facilement d'évaluer si le rythme est régulier ou irrégulier, c'est à dire si l'intervalle entre deux ondes R ou P est constant ou non. Pour ce contrôle on utilise un autre outil, qui se compose essentiellement d'un petit compas. Ce compas est ouvert, par exemple, entre deux ondes R (ou P) suivantes, puis positionné manuellement entre des paires d'ondes successives R (ou P), en observant visuellement si les extrémités du compas correspondent aux ondes. Cela permet d'identifier éventuelles fluctuations. Ledit compas, en revanche, ne fournit pas directement une valeur absolue de la fréquence, sauf si on le place sur une échelle graduée appropriée. Parfois, le compas est fourni avec un boîtier en plastique qui contient une échelle graduée de référence, mais l'utilisation n'est pas immédiate à cause de la nécessité d'ouvrir le compas sur le papier millimétré, puis le placer pour la lecture sur cette échelle de référence. En outre, il ya encore les inconvénients d'incertitude et de marge d'erreur qui ont été mentionnés ci-dessus.

Ont a proposé comme alternative des systèmes entièrement électroniques pour effectuer l'ECG, qui peuvent résoudre ces problèmes, mais ils ont l'inconvénient d'un coût élevé. Il y a, cependant, la nécessité d'une outil simple et à bon marché pour lire les ECG sur papier, qui sont encore très répandues.

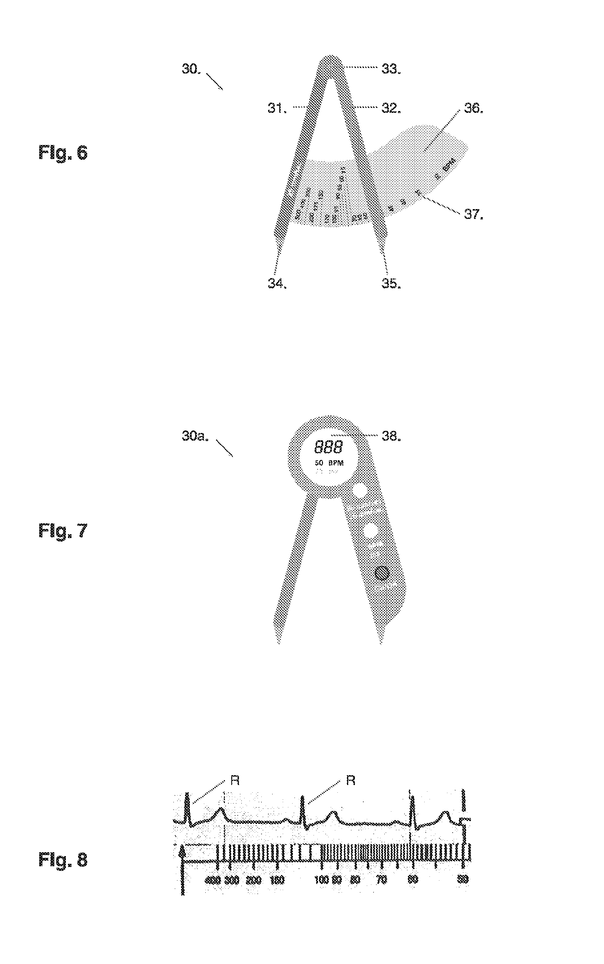

La Figure 8, par exemple, montre un tracé ECG typique. Une réglette selon l'art connue est placée avec la référence (flèche gauche) à un point situé sur le tracé, par exemple, le début de l'onde R; en observant l'échelle graduée, on peut déterminer que la prochaine onde R se produit en correspondance de la marque des 120 bpm, ce qui est la valeur approximative de la fréquence. On peut voir que cette méthode de mesure est inévitablement affectée par une approximation et comporte une marge d'erreur qui n'est pas négligeable. L'erreur a tendance à augmenter aux plus hautes fréquences où, comme on voit sur la Figure 8, l'échelle est beaucoup plus dense.

En résumé, l'art connue ne fournit pas un aide satisfaisant pour la lecture d'un graphique ECG. Le médecin doit souvent effectuer des calculs manuels ou utiliser des réglettes difficiles à lire, avec des échelles très denses et avec une marge d'erreur qui n'est pas négligeable, surtout aux hautes fréquences; des outils comme le compas, à son tour, présentent une comparaison entre un intervalle et l'autre, mais pas la valeur absolue, ni pour le temps ni pour les fréquences.

### Exposé de l'invention

Le problème de base de l'invention est de fournir un outil pour la lecture d'un ECG, qui pallie les inconvénients énumérés ci-dessus.

Le problème est résolu avec un outil pour lire un ECG, caractérisé en ce qu'il comprend:
- un premier élément de référence et un deuxième élément de référence, associés à des parties de l'instrument qui puissent être déplacées, de sorte que la distance linéaire entre le premier élément de référence et le deuxième élément de référence est réglable;
- un système de mesure intégré pour permettre la lecture d'au moins une grandeur caractéristique de l'ECG, en fonction de la distance entre les dits premier et deuxième élément de référence.

Le dit système de mesure peut être à lecture analogique, sur une échelle graduée, ou numérique sur un écran dédié. Des réalisations de l'invention sont possibles avec double lecture analogiques et numérique. Dans certaines réalisations ledit système de mesure comprend au moins une échelle graduée associée à l'un desdits premier et deuxième composants de l'outil. L'outil, compatible avec la taille, peut inclure une échelle ou une pluralité d'échelles graduées, pour plusieurs variables (par exemple la fréquence, temps) ou la même grandeur liée à différentes vitesses d'impression. Dans d'autres réalisations, ledit système de mesure comporte un appareil à lecture numérique qui peut être lu à partir de ladite au moins une taille caractéristique du tracé ECG. L'outil numérique peut prendre en charge ou remplacer l'échelle graduée.

Un système de mesure numérique peut comprendre un capteur tel qu'un codeur capacitif/inductif, optique, magnétique ou potentiomètre, pour fournir un signal lié à la distance entre le premier et le deuxième élément de référence, et l'outil de lecture numérique traite le signal pour le calcul d'au moins une grandeur caractéristique.

Dans un premier mode de réalisation préférée, l'outil est essentiellement conçu comme un pied à coulisse, numérique ou conventionnel. Le premier et le deuxième élément de référence, donc, sont représentés par les deux becs ou extrémités du pied à coulisse associés respectivement par le corps du pied coulisse lui-même. Dans un deuxième mode de réalisation préférée, l'outil est essentiellement conçue comme un compas, conventionnel ou avec affichage numérique. Dans ce cas la première et deuxième référence, sont représentés par les extrémités des tiges du compas.

Ultérieures caractéristiques sont les suivantes. Le système de mesure intégré peut comprendre une ou plusieurs échelles reportées directement sur le corps de l'instrument. Par exemple le pied à coulisse peut avoir une échelle normalisée pour une vitesse d'impression de 25 mm/s, et/ou une échelle pour 50 mm/s. Les grandeurs caractéristiques du tracé ECG, mesurables avec l'instrument selon l'invention, peuvent être représentées en bpm (fréquence cardiaque) ou en ms (temps) entre deux ondes, par exemple entre deux ondes R ou P. Dans ce cas, il faut noter que l'échelle de l'instrument n'est pas linéaire comme pour un pied à coulisse conventionnel pour des mesures de longueur, mais calculé sur la base de la formule qui permet de passer de la distance à la fréquence ou au temps selon l'échelle sélectionnée. L'instrument peut toutefois être réalisé pour la mesure de quelconque autre grandeur caractéristique d'un ECG.

Pour la version pied coulisse numérique, le système de mesure intégré peut comprendre un écran qui indique la valeur d'une grandeur caractéristique, par exemple la fréquence et/ou le temps entre deux ondes successives. Dans une réalisation l'outil permet de sélectionner l'échelle (25 ou 50 mm/s par exemple) et la grandeur à mesurer grâce à des touches de sélection. La mesure, en cette forme de réalisation, peut être conçue grâce à n'importe quel système déjà présent sur le marché (crémaillère, règle optique, codeur magnétique, codeur inductif,...). L'instrument calcule automatiquement la valeur à afficher.

Un instrument selon l'invention, en forme de compas analogique, comprend par exemple une réglette intégrée qui permet la lecture d'au moins une grandeur caractéristique, par exemple la fréquence en fonction de l'angle d'ouverture, de façon a surmonter les inconvénients des compas et des réglettes connus. Dans la réalisation du compas numérique, la mesure d'au moins une grandeur caractéristique peut être obtenue par un capteur et affichée sur écran. Le capteur est de type standard en fonction de la précision exigée, par exemple un codeur capacitif/inductif, optique, magnétique, potentiomètre, ou autre technologie déjà connue.

Le grand avantage de l'invention est que toutes les fonctions nécessaires pur la mesure des grandeurs d'un tracé ECG sont réunies en un unique instrument, facile à utiliser. Le coût de l'instrument analogique est très faible et l'instrument réunis en lui-même les prérogatives des réglettes et des compas utilisés jusqu'à présent ; l'instrument numérique permet une lecture encore plus simplifiée et permet aussi le passage d'une lecture d'une grandeur à l'autre, ou d'une échelle à l'autre, simplement en pressant une touche, bien en étant réalisé avec des components déjà connus avec prix assez bas.

Les caractéristiques et les avantages de l'invention seront plus claires avec la suivante description détaillée et à l'aide des figures annexées.

### Description des figures

Les Fig. de 1 a 5 illustrent des réalisations de l'invention de l'instrument selon l'invention, en forme de pied coulisse analogique ou numérique.

Les Fig. 6 et 7 illustrent des de réalisation de l'invention, en forme de compas.

### Description détaillée d'une méthode de réalisation préférée

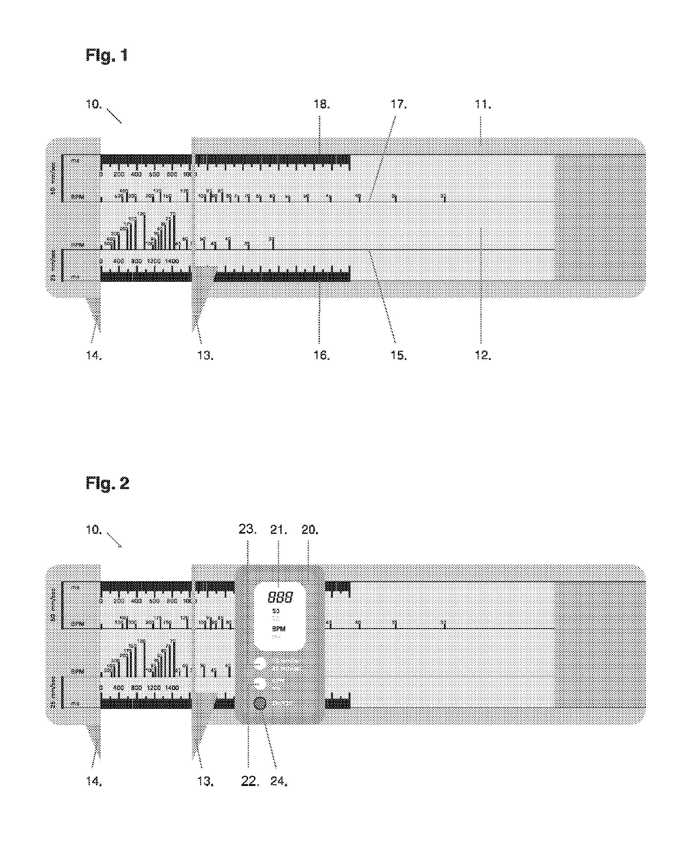

La Fig. 1 montre une première réalisation de l'invention, dans la quelle l'instrument 10 pour la lecture d'un tracé ECG est conçu comme un pied à coulisse, comprenant un corps 11 et un curseur coulissant 12, respectivement avec tiges ou becs 13,14. Un entre le corps 11 et le curseur 12 comporte au moins une échelle qui permet de déterminer une valeur de fréquence cardiaque (bpm) et/ou un intervalle de temps (ms), en fonction de l'écartement entre les becs 13 et 14. Dans l'exemple de la Fig.1, sur le curseur 12 sont reportées des échelles de lecture 15, 16, qui indiquent la fréquence et l'intervalle de temps et qui sont normalisées pour des vitesses d'impression de 25 mm/s, ainsi que des échelles 17, 18 qui sont normalisées à 50 mm/s.

La Fig. 2 montre une variante de l'instrument 10 qui comprend en outre un outil de lecture numérique 20. Cet instrument comprend un écran alphanumérique 21 qui peut illustrer la valeur exacte de la fréquence ou de l'intervalle de temps; les deux valeurs sont sélectionnées par une touche 22. Une autre touche 23 permet de sélectionner la vitesse d'impression, parmi un numéro présélectionnée (normalement 25 mm/s ou 50 mm/s). Un dernier bouton 24 permet la mise en marche de l'appareil.

Le relevé de la distance entre les becs 13 et 14, dans le cas de Fig. 2, est réalisé avec la technique usuelle des calibres digitaux. L'instrument 20, entre autre, réalise le calcul de transformation entre la mesure de la distance des becs (mm) et la mesure de fréquence ou respectivement de temps, selon les sélections des touches 22, 23.

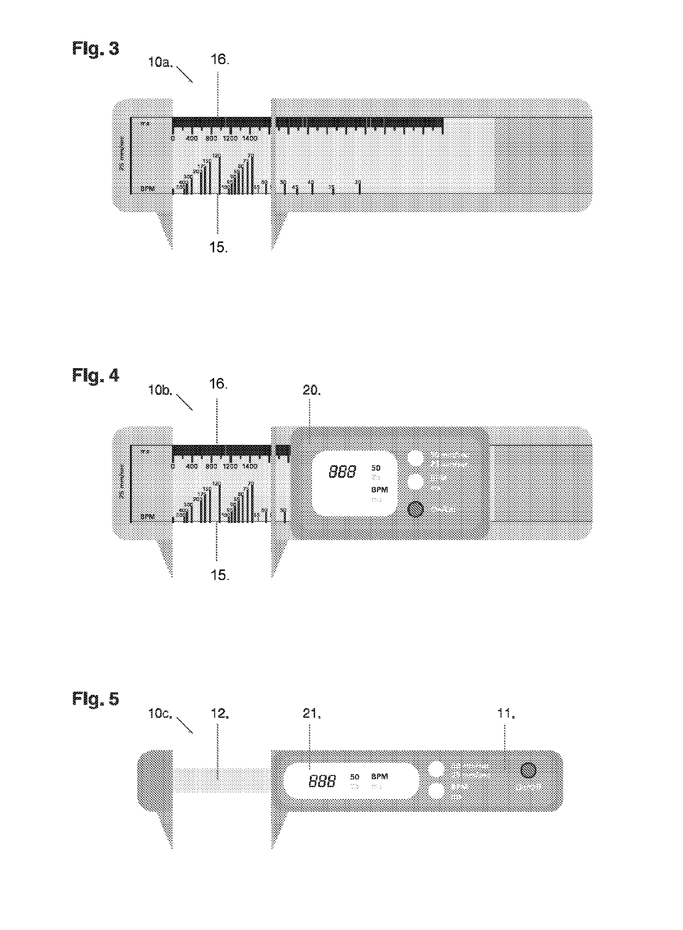

Les Fig. 3, 4 et 5 illustrent des réalisations dans lesquelles, respectivement, un instrument à pied à coulisse 10a, 10b, 10c comprend seulement une échelle graduée, une échelle graduée et un instrument numérique, ou seulement l'instrument numérique. D'autres réalisations équivalentes sont clairement possible dans le domaine de l'invention. L'instrument selon l'invention peut être exclusivement analogique (Fig.1 et 3), analogique/ numérique (Fig. 2 et 4) ou purement numérique (Fig.5). Dans la version Fig.5 l'écran 21 et les boutons 22-24 sont intégrés dans le corps 11.

Une réalisation à compas est démontrée par la Fig. 6. L'instrument 30 comprend essentiellement deux tiges 31, 32 articulées sur un pivot 33. Les extrémités 34, 35 correspondent aux éléments de référence. L'instrument est doté d'un support 36, de préférence en forme d'arc et fixé à l'une des deux tiges, par exemple la tige 31, qui comporte une échelle graduée 37 pour la lecture d'une caractéristique spécifique, comme la fréquence en bpm en fonction de l'ouverture du compas, donc de la distance linéaire, sur un plan d'appui, entre les références 34 et 35. Le support 36 peut comporter, compatible avec les dimensions, plusieurs échelles graduées et se référer à différentes grandeurs (par exemple bpm et intervalles de temps) et/ou à la même grandeur normalisée pour des vitesses différentes (par exemple 25 / 50 mm/s), comme démontrée dans les Fig.1 et 2.

La Fig.7 montre une variante de l'instrument à compas 30a dans laquelle la mesure de l'angle et donc de la grandeur caractéristique, par exemple de la fréquence et du temps, est réalisée au moyen d'un capteur et visualisée grâce à un écran 38. Le capteur est de type connue dans l'état de la technique et peut être par exemple un codeur capacitif/inductif, optique, magnétique, potentiomètre ou autre. L'instrument 30a présente des boutons de mise en marche et de sélection analogue à ceux décrits pour la réalisation de la Fig. 2.

L'utilisation de l'invention est la suivante: à titre d'exemple, on se réfère à la mesure de la fréquence cardiaque sur un tracé ECG, entre deux ondes R. L'instrument de type analogique (Fig. 1, 3 et 6), est positionné avec le premier élément de référence, respectivement 13 ou 34, en correspondance du pic de l'onde R; l'instrument vient ainsi «ouvert» amenant l'autre élément de référence, 14 ou 35, en correspondance du pic de l'onde R suivante. L'échelle graduée 15, ou l'échelle 37 sur le support 36, permet le lire avec précision la fréquence. L'utilisation de l'instrument numérique (Fig. 2, 4, 5 et 7) prévoit la mise en marche, la sélection de l'échelle de vitesse et/ou du paramètre à visualiser, et donc le positionnement sur le tracé ECG de façon sensiblement équivalente comme décrit ci-dessus.

## Revendications

1. Instrument pour la lecture d'un tracé ECG imprimé, ledit instrument étant caractérisé du fait d'inclure:
- un premier élément de référence (13, 34) et un deuxième élément de référence (14, 35) associés respectivement à un premier composant (11, 31) et à un deuxième composant (12, 32) de l'instrument, déplaçables l'un par rapport à l'autre de façon que la distance linéaire entre le premier et le deuxième élément de référence soit réglable;
- un système de mesure intégré pour permettre la lecture d'au moins une grandeur caractéristique d'un tracé ECG, qui dépend de la distance entre ledit premier et ledit deuxième élément de référence.

2. Instrument selon la revendication 1, dans lequel ledit système de mesure comprends au moins une échelle de lecture graduée (15, 16 , 37) associée à un desdits premier et deuxième composant de l'instrument.

3. Instrument selon la revendication 1 ou 2, dans lequel ledit système de mesure comprends un système de lecture numérique (20, 38) qui permet la lecture d'au moins une grandeur caractéristique du tracé ECG.

4. Instrument selon la revendication 3, comprenant un capteur comme par exemple un codeur capacitif/inductif, optique, magnétique ou potentiométrique, capable de fournir un signal qui soit en corrélation à la distance entre le premier et le deuxième élément de référence, et ce signal est élaboré par ledit instrument de lecture numérique pour calculer au moins une des grandeurs caractéristiques du tracé ECG.

5. Instrument selon la revendication 3, comprenant une commande (22) qui permet de visualiser sur écran numérique une grandeur sélectionnée parmi une pluralité de grandeurs prédéterminées.

6. Instrument selon une quelconque des revendications de 1 à 5, ledit instrument étant réalisé comme un pied à coulisse, qui comprend un corps (11) et un curseur (12) coulissant par rapport au dit corps, et deux tiges ou becs (13, 14) qui représentent respectivement ledit premier et ledit deuxième élément de référence.

7. Instrument selon une quelconque des revendications de 1 à 5, ledit instrument (30) réalisé comme un compas avec deux tiges (31, 32) reliées entre elles par un pivot (33), où les extrémités desdites tiges correspondent au dit premier et au dit deuxième élément de référence.
